# EUROPEAN PATENT SPECIFICATION

(11) **EP 1 687 011 B1**
(45) Date of publication and mention of the grant of the patent: **16.07.2008**
(21) Application number: 04768807.2
(22) Date of filing: 08.10.2004
(51) Int. Cl.: A61K 35/74, A61K 39/095

(54) **MODIFIED WHOLE CELL, CELL EXTRACT AND OMV-BASED VACCINES**
MODIFIZIERTE GANZZELL-, ZELLEXTRAKT- UND OMV-BASIERTE IMPFSTOFFE
CELLULE ENTIERE MODIFIEE, EXTRAIT DE CELLULE ET VACCINS A OMV

(30) Priority: 09.10.2003 GB 0323709
(43) Date of publication of application: 09.08.2006
(73) Proprietor: Health Protection Agency, Salisbury, Wiltshire SP4 0JG (GB)
(72) Inventor: GORRINGE, Andrew R., Health Protection Agency, Salisbury, Wiltshire SP4 0JG (GB); REDDIN, Karen M., Health Protection Agency, Salisbury, Wiltshire SP4 0JG (GB); GRAY-OWEN, Scott D., Oakville, Ontario L6J 7E7 (CA); BOULTON, Ian C., Toronto, Ontario, M6K 2C8 (CA)
(74) Representative: Naylor, Kathryn May
(86) International application number: PCT/GB2004/004274
(87) International publication number: WO 2005/035733

(56) References cited:
- WO-A-03/051379
- WO-A-20/04014417
- US-A1- 2003 059 444
- PEETERS C C A M ET AL: "Phase I clinical trial with a hexavalent PorA containing meningococcal outer membrane vesicle vaccine" VACCINE, BUTTERWORTH SCIENTIFIC. GUILDFORD, GB, vol. 14, no. 10, July 1996 (1996-07), pages 1009-1015, XP004057633 ISSN: 0264-410X
- BOULTON IAN C ET AL: "Neisserial binding to CEACAM1 arrests the activation and proliferation of CD4+ T lymphocytes." NATURE IMMUNOLOGY. MAR 2002, vol. 3, no. 3, March 2002 (2002-03), pages 229-236, XP002323222 ISSN: 1529-2908
- COHEN M S ET AL: "Human experimentation with Neisseria gonorrhoeae: progress and goals." THE JOURNAL OF INFECTIOUS DISEASES. MAR 1999, vol. 179 Suppl 2, March 1999 (1999-03), pages S375-S379, XP002323375 ISSN: 0022-1899
- VAN DER LEY P ET AL: "Construction of Neisseria meningitidis strains carrying multiple chromosomal copies of the porA gene for use in the production of a multivalent outer membrane vesicle vaccine" VACCINE, BUTTERWORTH SCIENTIFIC. GUILDFORD, GB, vol. 13, no. 4, 1995, pages 401-407, XP004057740 ISSN: 0264-410X
- LEY VAN DER P ET AL: "CONSTRUCTION OF A MULTIVALENT MENINGOCOCCAL VACCINE STRAIN BASED ONTHE CLASS 1 OUTER MEMBRANE PROTEIN" INFECTION AND IMMUNITY, AMERICAN SOCIETY FOR MICROBIOLOGY. WASHINGTON, US, vol. 60, no. 8, August 1992 (1992-08), pages 3156-3161, XP000946454 ISSN: 0019-9567
- WIERTZ E J ET AL: "T-cell responses to outer membrane proteins of Neisseria meningitidis: comparative study of the Opa, Opc, and PorA proteins" INFECTION AND IMMUNITY, AMERICAN SOCIETY FOR MICROBIOLOGY. WASHINGTON, US, vol. 64, no. 1, January 1996 (1996-01), pages 298-304, XP002127427 ISSN: 0019-9567
- WEDEGE ELISABETH ET AL: "Antibody specificities and effect of meningococcal carriage in icelandic teenagers receiving the Norwegian serogroup B outer membrane vesicle vaccine." INFECTION AND IMMUNITY. JUL 2003, vol. 71, no. 7, July 2003 (2003-07), pages 3775-3781, XP002322410 ISSN: 0019-9567
- CLAASSEN I ET AL: "Production, characterization and control of a Neisseria meningitidis hexavalent class 1 outer membrane protein containing vesicle vaccine" VACCINE, BUTTERWORTH SCIENTIFIC. GUILDFORD, GB, vol. 14, no. 10, July 1996 (1996-07), pages 1001-1008, XP004057632 ISSN: 0264-410X
- NORMARK STAFFAN ET AL: "Gonococci cause immunosuppression by engaging a coinhibitory receptor on T lymphocytes." NATURE IMMUNOLOGY. MAR 2002, vol. 3, no. 3, March 2002 (2002-03), pages 210-211, XP002323223 ISSN: 1529-2908
- GRAY-OWEN S D ET AL: "CD66 carcinoembryonic antigens mediate interactions between Opa-expressing Neisseria gonorrhoeae and human polymorphonuclear phagocytes." THE EMBO JOURNAL. JUN 1997, vol. 16, no. 12, June 1997 (1997-06), pages 3435-3445, XP002323383 ISSN: 0261-4189
- KUPSCH E M ET AL: "Variable opacity (Opa) outer membrane proteins account for the cell tropisms displayed by Neisseria gonorrhoeae for human leukocytes and epithelial cells." THE EMBO JOURNAL. FEB 1993, vol. 12, no. 2, February 1993 (1993-02), pages 641-650, XP008045296 ISSN: 0261-4189
- DEHIO C ET AL: "The role of neisserial Opa proteins in interactions with host cells." TRENDS IN MICROBIOLOGY. DEC 1998, vol. 6, no. 12, December 1998 (1998-12), pages 489-495, XP002340390 ISSN: 0966-842X
- GRANT C C ET AL: "Proteoglycan receptor binding by Neisseria gonorrhoeae MS11 is determined by the HV-1 region of OpaA." MOLECULAR MICROBIOLOGY. APR 1999, vol. 32, no. 2, April 1999 (1999-04), pages 233-242, XP002340391 ISSN: 0950-382X
- VAN PUTTEN J P ET AL: "Binding of syndecan-like cell surface proteoglycan receptors is required for Neisseria gonorrhoeae entry into human mucosal cells." THE EMBO JOURNAL. 15 MAY 1995, vol. 14, no. 10, 15 May 1995 (1995-05-15), pages 2144-2154, XP002340392 ISSN: 0261-4189
- GRAY-OWEN S D ET AL: "Differential Opa specificities for CD66 receptors influence tissue interactions and cellular response to Neisseria gonorrhoeae." MOLECULAR MICROBIOLOGY. DEC 1997, vol. 26, no. 5, December 1997 (1997-12), pages 971-980, XP002340393 ISSN: 0950-382X
- BOS M P ET AL: "Carcinoembryonic antigen family receptor recognition by gonococcal Opa proteins requires distinct combinations of hypervariable Opa protein domains" INFECTION AND IMMUNITY, AMERICAN SOCIETY FOR MICROBIOLOGY. WASHINGTON, US, vol. 70, no. 4, April 2002 (2002-04), pages 1715-1723, XP002322409 ISSN: 0019-9567
- BILLKER O ET AL: "The structural basis of CEACAM-receptor targeting by neisserial Opa proteins." TRENDS IN MICROBIOLOGY. JUN 2000, vol. 8, no. 6, June 2000 (2000-06), pages 258-260 ; dis, XP002340394 ISSN: 0966-842X
- BOS M P ET AL: "Differential recognition of members of the carcinoembryonic antigen family by Opa variants of Neisseria gonorrhoeae." INFECTION AND IMMUNITY. JUN 1997, vol. 65, no. 6, June 1997 (1997-06), pages 2353-2361, XP002340395 ISSN: 0019-9567
- DE JONGE MARIEN I ET AL: "Conformational analysis of opacity proteins from Neisseria meningitidis." EUROPEAN JOURNAL OF BIOCHEMISTRY / FEBS. NOV 2002, vol. 269, no. 21, November 2002 (2002-11), pages 5215-5223, XP002340396 ISSN: 0014-2956
- VIRJI M ET AL: "The N-domain of the human CD66a adhesion molecule is a target for Opa proteins of Neisseria meningitidis and Neisseria gonorrhoeae." MOLECULAR MICROBIOLOGY. DEC 1996, vol. 22, no. 5, December 1996 (1996-12), pages 929-939, XP002340397 ISSN: 0950-382X

## Description

The invention relates to modified whole cell, cell extract and OMV-based compositions and components thereof for treatment or prevention of disease by Gram negative bacteria, in particular disease caused by *Neisseria.*

A significant number of human and animal pathogens fall within the Gram negative classification of bacteria, including members of the genera *Neisseria, Moraxella, Kingella, Acinetobacter, Brucella, Bordetella, Haemophilus, Escherichia, Chlamydia, Legionella, Pseudomonas, Proteus* and *Yersinia. Neisseria meningitidis* (the meningococcus) is the organism that causes meningococcal meningitis and is of particular importance as a worldwide health problem. In many countries the incidence of this disease is increasing. *N. meningitidis* is also responsible for meningococcal septicaemia, which is associated with rapid onset of symptoms and high mortality, with around 22% of cases proving fatal. Other Gram negative bacteria cause a range of human infections including meningitis (*H*. *influenzae*), plague (*Y. pestis*), gastroenteritis (*E. coli*), venereal disease (*N. gonorrhoeae*) and nosocomial infection (*P. aeruginosa*).

It is desirable to provide broad spectrum vaccines that provide protective immunity in animals, particularly humans, against Gram negative bacterial infection.

Outer membrane vesicles (OMVs) derived from the human pathogen *Neisseria meningitidis* are currently utilized as a source of antigen for a protective meningococcal vaccine.

To address the difficulties associated with achieving broad spectrum protection researchers have attempted to "enrich" OMVs with particular antigens that might enhance the immunogenic potential of the OMV. In WO-A-00/25811 OMVs isolated from *N. meningitidis* are combined with heterologous antigens, e.g. Transferrin binding protein (Tbp), or a genetically modified *N. meningitidis* expresses such antigens recombinantly and antigen enriched OMVs are derived therefrom. A similar approach was adopted in WO-A-01/09350 which describes vaccine compositions comprising OMVs from *N. meningitidis, M. catarrhalis* and *H. influenzae.* In certain embodiments these organisms have been genetically modified to overexpress particular immunogenic moieties.

A difficulty with such OMV-based vaccines is that, to achieve adequate protection, vaccines have to be administered at frequent intervals, or boosters have to be given to maintain a protective immune response.

Despite the availability of effective antibiotic therapies to combat infection, *Neisseria gonorrhoeae* causes about 78 million infections globally per annum. Gonorrhoea is characterized by an intense inflammatory response that leads to the liberation of large amounts of urethral or cervical pus, consisting primarily of neutrophils with extracellular and intracellular-associated *N. gonorrhoeae.* Up to 15% of infected men and 80% of infected women remain asymptomatic. In such situations, infection tends to be prolonged and is consistently transmissible, both vertically (to neonates of infected mothers) and horizontally (to sexual partners). If undetected, such infections are a source of significant morbidity, including severe conjunctivitis in neonates, disseminated gonococcal infection, pelvic inflammatory disease and sterility through fallopian tube scarring.

The persistence of *N. gonorrhoeae* within the population relies on the fact that gonorrhoea can be contracted repeatedly, and there is little evidence that the exposure to or colonization by this organism reduces an individual's susceptibility to subsequent infection. This is at least partially attributable to the antigenic variation of gonococcal surface epitopes, however, individuals can be reinfected by the same serotype of *N. gonorrhoeae* indicating that immune evasion is not the only survival strategy used by this pathogen.

It is hence desired to provide improved vaccination against initial or repeat gonococcal infection.

The colony Opacity (Opa) proteins expressed by *Neisserial* species are an important virulence determinant. Boulton et al: Nat Immunol. 2002 Mar;3(3):229-36 describe how, *in vitro,* Opa proteins suppressed T lymphocyte activation and proliferation. Opa proteins are described across the literature as ideal vaccine targets. Wiertz et al (Infect Immun. 1996 Jan;64(1):298-304.) and De Jonge et al (Infect Immun. 2003 May;71(5):2331-40.) identify Opa-antibodies as important in protection against gonococcal infection. De Jonge *et al* noted how Opa-antibodies reduced *Neisserial* adhesion and so propose including Opa in vaccination. Schneider et al J Infect Dis. 1995 Jul;172(1):180-5.) propose use of an Opa-based vaccine against gonorrhoea. Plummer et al (J. Clin. Invest. 1994; 93: 1748-1755) correlated antibodies to Opa with reduced risk of gonococcal salpingitis and promote Opa-based vaccines. Boulton et al Nature immunol. 2002, vol 3 pp 229 to 236 and Normark Staffan et al Nature Immunol, 2002, vol 3 no 3 pp 210 to 211 discuss the interaction of Opa and *CEACAM1* in *Neisseria gonorrhoeae.*

An object of the present invention is to provide microorganisms, compositions and vaccines, including OMV-based vaccines for use in treatment or prevention of disease by Gram negative bacteria. An object of specific objects of the invention is to provide such a vaccine that solves or at least ameliorates problems associated with current vaccination against meningococcal disease and gonorrhoea.

In a first aspect, the present invention provides use of a composition comprising *Neisseria* outer membrane vesicles which contain Opa that does not bind to *CEACAM1* and which are substantially free of Opa that binds *CEACAM1* for the manufacture of a vaccine for treatment or prevention of meningococcal disease, wherein the outer membrane vesicles are from *Neisseria* that have been modified by mutation to express an Opa that does not bind to *CEACAM1.*

Accordingly, the invention relates to microorganisms, compositions, vaccines, components of vaccines, methods of obtaining the aforementioned and genes encoding the aforementioned, substantially free of Opa that binds *CEACAM1.* These are suitable for use in treatment or prevention of disease caused by Gram negative bacteria. The invention also relates to microorganisms, compositions, vaccines, components of vaccines, methods of obtaining the aforementioned and genes encoding the aforementioned that are suitable for treatment or prevention of meningococcal disease or gonococcal disease and are substantially free of protein that suppresses activation or proliferation of a CD4+ T cell.

The microorganisms are typically Gram negative bacteria, namely. *Neisseria,* which are selected to be substantially free of Opa that binds *CEACAM1* or that are modified so as to be substantially free of Opa that binds *CEACAM1.* They are modified by mutation to express an Opa that does not bind *CEACAM1.* Compositions of the invention contain such bacteria or immunogenic extracts thereof, for example protein preparations thereof. Vaccines comprising the microorganisms or extracts thereof may contain live bacteria, live attenuated bacteria or dead bacteria. Generally, hereafter, reference to compositions of the invention is intended to refer to all of microorganisms, compositions, vaccines, and components of vaccines unless otherwise indicated.

The invention also relates to a method of selecting a microorganism, composition, vaccine or vaccine component, for use in treatment or prevention of disease caused by Gram negative bacteria, the method comprising determining whether said microorganism, composition, vaccine or vaccine component is substantially free of Opa that binds *CEACAM1.*

A further method related to the invention is one for selecting a microorganism, composition, vaccine or vaccine component, for use in treatment or prevention of meningococcal disease or gonococcal disease, comprising determining whether said microorganism, composition, vaccine or vaccine component is substantially free of protein that suppresses activation or proliferation of a CD4+ T cell.

Said microorganism, composition, vaccine or vaccine component is preferably substantially free of Opa that binds *CEACAM1* or is modified so as to be substantially free of Opa that binds *CEACAM1.* In a preferred method a *Neisseria* is selected to be substantially free of Opa that binds *CEACAM1* or is modified, such as by mutation as described in more detail below, so as to be substantially free of Opa that binds *CEACAM1.*

A population of Gram negative bacteria disclosed herein, being 1,000 or more in number, is substantially free of bacteria expressing Opa that binds *CEACAM1.* Compositions are obtained therefrom which likewise are substantially free of Opa that binds *CEACAM1.*

In another embodiment, the present application discloses a composition, comprising Gram negative bacteria outer membrane vesicles, preferably *Neisseria* outer membrane vesicles, wherein the vesicles are substantially free of Opa.

The Opa content of the vesicles is preferably reduced by at least a factor of 10 compared with the Opa content of OMVs obtained from normal *Neisseria,* the benchmark for normal *Neisseria* being the Opa content of OMVs obtained from *N. meningitidis* strain K454, and more especially the Opa represents 0.5% or less by weight of the total protein content of OMVs.

The compositions of the invention comprise outer membrane vesicles, wherein the vesicles comprise an Opa protein that does not bind to *CEACAM1.* These vesicles are in addition substantially free of Opa that binds *CEACAM1.*

Also disclosed herein is a composition comprising outer membrane vesicles, wherein the vesicles comprise a protein which is antigenic, elicits production of antibodies which bind to Opa, and does not bind to *CEACAM1.* The protein may be a mutant or variant or fragment or derivative or mimic of Opa.

A yet further composition disclosed herein comprises outer membrane vesicles, wherein the vesicles comprise an antagonist which inhibits binding of Opa to *CEACAM1.*

The term "Opa" refers to a Gram negative, especially *Neisserial,* colony opacity associated protein that can modulate or suppress an immune response or inhibit tumor growth. Preferably, the Opa protein can bind to *CEACAM1* and cause ligation of *CEACAM1* with consequent immune suppression or inhibition of an immune cell.

Specific Opa proteins include Opa₅₂ and Opa₅₇. Since the neisserial Opa proteins are highly antigenically variable, the Opa protein may be any of the Opa proteins that can be expressed by various neisserial species and that also bind to the *CEACAM1* receptor or to homologous non-human receptors. Opa proteins include the Opa proteins encoded by any neisserial species, including the pathogenic *Neisseria gonorrhoeae* and *Neisseria meningitidis* and the commensal species such as *Neisseria lactamica* and *Neisseria subflava,* for which their Opa proteins have been shown to bind *CEACAM1,* and other commensals that also express Opa proteins.

The term "Opa protein" also refers to analogous proteins from other bacterial species. This includes, but is not restricted to, the *CEACAM1*-binding proteins of *Haemophilus influenzae.* Like the *Neisserial* Opa proteins, *the H. influenzae* P5 proteins are antigenically variable outer membrane proteins that are predicted to form a beta-barrel structure with eight transmembrane regions and four extracellular loops. As with the Opa proteins, the P5 transmembrane regions and the 4th surface-exposed loop are well conserved, while the sequence within the other surface-exposed loops is variable. Also like various of the *Neisserial* Opa proteins, the *H. influenzae* P5 proteins function in attachment to host cells via binding to CEACAM receptors, including *CEACAM1.*

In the present invention, we provide compositions purposively lacking, or at least reduced in, *CEACAM1*-reactive Opa content. We also provide compositions containing mutants or variants or fragments or derivatives or mimics of Opa, which mutants, variants, fragments, mimics and derivatives do not activate *CEACAM1.* These compositions thus offer the basis of vaccines against disease, especially *Neisserial* disease, more especially meningococcal and gonococcal disease, with improved development of immune response and immune memory in patients. The mutants, variants, fragments and derivatives enable anti-Opa antibodies and other immune responses to be generated *in vivo* without the disadvantages of activation of *CEACAM1,* for example without immune suppression and/or reduction in immune memory.

Compositions of the invention are for use in treating patients, typically human patients, and the invention provides a method of treatment of an individual comprising administering a composition of the invention.

The invention also provides for the manufacture of compositions for vaccination and for vaccine components. Thus, in a further aspect, the present invention provides a method for manufacturing a medicament for treatment or prevention of meningococcal disease, the method comprising:-
(a) obtaining a *Neisseria;*
(b) determining whether the *Neisseria* expresses an Opa protein that binds to *CEACAM1;*
(c) if the *Neisseria* expresses an Opa protein that binds to *CEACAM1,* discarding the *Neisseria* and repeating steps (a) to (c);
(d) retaining the *Neisseria* if it expresses a mutant or variant or fragment or derivative of Opa, wherein the mutant or variant or fragment or derivative does not bind to *CEACAM1;*
(e) raising an antibody to the mutant or fragment or derivative;
(f) determining whether the antibody also binds to an Opa protein that binds to *CEACAM1;* and
(g) preparing a composition comprising the retained *Neisseria* of step (d).

Also disclosed herein is a method for preparing a composition for use as or in manufacture of a vaccine, comprises:-
(a) obtaining a Gram negative bacterium;
(b) determining whether the bacterium expresses an Opa protein that binds to *CEACAM1;*
(c) if the bacterium expresses the Opa protein, discarding the bacterium and repeating steps (a) to (c);
(d) retaining the bacterium if it does not express the Opa protein; and
(e) preparing a composition comprising the retained bacterium of step (d).

In this invention the bacterium is a *Neisseria.*

The bacterium may be one that does not express *CEACAM1*-reactive Opa, but does produce non-functional Opa. The methods of the invention include a further step of retaining a bacterium which expresses a mutant or variant or fragment or derivative or mimic of Opa, wherein the mutant or variant or fragment or derivative or mimic does not bind to *CEACAM1.*

Such mutants or variants or fragments or derivatives or mimics of Opa may arise naturally in the bacterial population. A preferred method of the invention, however, is one that induces production of such proteins, and comprises:-
(a) obtaining a *Neisseria;*
(b) carrying out mutagenesis on the *Neisseria;*
(c) determining whether the *Neisseria* expresses a mutant or fragment or variant or derivative or mimic of an Opa protein that does not bind to *CEACAM1;*
(d) isolating the mutant or variant or fragment or derivative or mimic; wherein the mutant or variant or fragment or derivative does not bind to *CEACAM1.*

*Neisseria* species are naturally competent and amenable to mutagenesis via recombination between homologous DNA sequences and, further, the availability of the entire meningococcal and gonococcal genome sequences enable accurate determination of suitable sites for mutagenesis.

One method of creating or obtaining a bacterium of the invention is to clone an Opa gene, insert the cloned gene into an expression vector, and mutagenise the cloned gene. The cloned gene can then be expressed and its product tested for (i) binding to *CEACAM1,* and (ii) generating antibodies that bind to native Opa. A mutated gene which produces a product having desired properties can then be expressed *in vitro* to obtain a mutant protein for the invention. The protein can be incorporated into compositions, especially vaccines. This technique is also suitable for generating a fragment of Opa which likewise can be tested for the desired properties. The mutated gene can be inserted into a host bacterium, preferably a *Neisseria,* to replace a native *CEACAM1* binding Opa.

Transposons (Tns) are widely used for mutagenesis where available. This is because 1) it is easy to map the site of mutation (insertion of the transposon) by sequencing out from the ends of the transposon; 2) transposons can be used which insert only once in the chromosome, allowing analysis of a mutant phenotype resulting from a single insertion mutation; 3) existing procedures allow simultaneous screening of large numbers of potentially interesting mutants. Other classical methods for mutagenesis include the use of UV light and, more frequently, the use of mutagenic agents to introduce physical changes in the DNA which results in the mutation of genes. The mutations introduced by such methods are far more random than those generated by Tn since individual base pairs are the target (typically G:C-> A:T transitions). There is no direct requirement for complex genetic systems, as with Tn mutagenesis using these approaches, however vectors may be required to identify the site of mutation by complementation. Typically one establishes a dose vs. survival curve for the agent then uses the dose which kills approximately 90% of the population to ensure mutations are introduced. A more detailed protocol is given in the Examples for EMS and NTG mutagenesis.

It is desirable for the compositions of the invention, including OMV-based vaccines, to include antigens that will induce protective antibodies that bind to Opa *in vivo*. Hence, methods of the inventions for generating and identifying such antigens typically also include the steps of:-
(e) raising an antibody to the mutant or fragment or variant or derivative; and
(f) determining whether the antibody also binds to an Opa protein that binds to *CEACAM1.*

Also disclosed herein is an isolated mutant or variant or fragment or derivative or mimic of Opa, wherein the mutant or variant or fragment or derivative or mimic does not bind to *CEACAM1,* preferably as obtained according to the methods described herein.

OMV-based vaccines exist at present, using OMVs from various *Neisserial* species. Such vaccines may contain Opa protein that binds to *CEACAM1,* though the consequences of this have until now been unappreciated. Also disclosed herein is a method of manufacture or testing of a vaccine, the method comprising:-
(a) obtaining a sample of a vaccine or of a component of a proposed vaccine against a Gram negative bacteria; and
(b) determining whether the sample contains an Opa protein that binds to *CEACAM1.*

Thus, it is now possible to screen new and existing vaccines to determine whether Opa content renders them suitable or unsuitable for therapeutic use. This information may also assist in explaining the different efficacies of respective vaccines in trials and commercial use.

A maximum level of Opa may be acceptable, and hence it is optional to determine the weight % of the Opa protein, if present, by weight % of total protein content in the vaccine or in the sample. The vaccine or the component may then be rejected if the sample contains the Opa protein, or if the weight % of the Opa protein is above a predetermined level, e.g. 0.5%.

The invention also relates to use of (a) *Neisseria* or (b) *Neisseria* outer membrane vesicles which (i) are substantially free of Opa, (ii) comprise an Opa protein that does not bind to *CEACAM1,* (iii) comprise a mutant or variant or fragment or derivative of Opa that does not bind to *CEACAM1,* or (iv) comprise an antagonist which inhibits binding of Opa to *CEACAM1,* in manufacture of a medicament for treatment or prevention of meningococcal or gonococcal disease with improved stimulation of immune memory or reduced inhibition of T cell function (e.g. activation and/or proliferation).

The invention applies in particular to OMVs from Gram negative bacteria, being those bacteria that fail to resist decolourisation in the commonly known Gram staining method. Gram negative bacteria are characterised by a complex multilayer cell wall and often possess an outer layer polysaccharide capsule - e.g. *N. meningitidis,* although in some species this capsule is absent - e.g. *N. lactamica.*

Outer membrane vesicles (OMVs), also referred to as blebs, are vesicles formed or derived from fragments of the outer membrane of a Gram negative bacterium. OMVs typically comprise outer membrane proteins (OMPs), lipids, phospholipids, periplasmic material and lipopolysaccharide (LPS). Gram negative bacteria, especially pathogens like *N*. *meningitidis,* often shed OMVs during virulent infections in a process known as blebbing. OMVs can also be obtained from Gram negative bacteria via a number of known chemical denaturation processes. Liposomes, comprising a lipid bilayer and typically enclosing an aqueous core, can be regarded for the purposes of the present invention as constituting a synthetic equivalent to OMVs, and embodiments of the invention described with reference to OMVs apply *mutatis mutandis* to embodiments carried out with and relating to liposomes.

A "vaccine" as referred to herein is defined as a pharmaceutical or therapeutic composition used to inoculate an animal in order to immunize the animal against infection by an organism, typically a pathogenic organism. A vaccine will typically comprise one or more antigens derived from one or more organisms which on administration to an animal will stimulate active immunity and protect that animal against infection with these or related pathogenic organisms.

Vaccine compositions that are formulated as pharmaceuticals will typically comprise a carrier. If in solution or in liquid aerosol suspension, suitable carriers can include saline solution, sucrose solution, or other pharmaceutically acceptable buffer solutions. An aerosol formulation will typically additionally comprise a surfactant. Alternatively, vaccine compositions include microencapsulated OMV compositions. Such microcapsules will generally comprise a biocompatible polymer shell or core, such as made from polylactide-co-glycolide (PLG). Vaccine compositions can additionally comprise an adjuvant, for example where administration is via the parenteral route. Suitable adjuvants include aluminium hydroxide.

Vaccines are suitably administered to an animal via a number of routes. For example, parenterally - e.g intramuscularly, trans-dermally - or via other routes - e.g. intra-nasally, orally, topically - or via any other commonly known administrative route.

Certain proteins can be recombinantly expressed in Gram negative bacteria and thereby enable enrichment or alteration of the antigenic profile of the bacterial outer membrane. Genetic modification of a bacterial source organism thereby allows for manipulation of the antigenic profile of OMVs that are obtained from these organisms. When proteins that are not normally present in the bacterial outer membrane, and thus in an OMV derived therefrom, are introduced via recombinant expression techniques, these "non-native" proteins and polypeptides are described as heterologous antigens. The contents ofWO-A-embodiments of the invention that the vaccine comprises OMVs rather than live attenuated or dead pathogenic organisms which can pose a greater risk of infection or adverse reaction.

Gram negative species include those selected from *Neisseria, Moraxella, Kingella, Acinetobacter, Brucella, Bordetella, Porphyromonas, Actinobacillus, Borelia, Serratia, Campylobacter, Helicobacter, Haemophilus, Escherichia, Legionella, Salmonella, Pseudomonas* and *Yersinia.* The invention is concerned with *Neisseria.*

Suitable methods for extracting OMVs from bacterial sources include deoxycholate extraction, Tris/HCl/EDTA extraction, and lithium acetate extraction. Protocols for performing such extractions are described in more detail in the literature. However, it will be appreciated by the skilled person that virtually any chemical and/or physical technique that enables disruption of the bacterial cell outer membrane in order to release sufficient OMVs for purification and isolation, is suitable for preparation of the compositions of the invention.

The invention also relates to methods of vaccinating animals, especially humans, against Gram negative bacterial infection utilising the compositions of the invention. In particular, the invention provides methods for vaccinating animals against meningococcal infection. Also provided are uses of the compositions of the invention in the vaccination of animals, including humans, against meningococcal infection. Also disclosed are uses of the compositions of the invention in the manufacture of vaccines for inoculating animals in order to stimulate protective immunity to Gram negative bacterial infection. OMVs are of use in mucosally administered compositions, as LPS toxicity is less and LPS can function as an adjuvant.

OMVs of the invention have reduced content of, or are free of, Opa proteins which recognize carcinoembryonic antigen-related cell adhesion molecule 1 (*CEACAM1*) and which suppress both the activation and proliferation of Jurkat CD4⁺ T lymphocytes in response to various stimuli *in vitro* and have similar effects on primary (non-malignant) cells of a similar type.

An advantage of the invention is that our OMVs avoid the presence of *CEACAM1*-reactive Opa and thereby, in comparison with OMVs that do contain *CEACAM1* - reactive Opa, show improved lymphocyte response to activating stimuli, decreased lymphocyte death, and by corollary, increased development of protective immunity. This is surprising as, given the species specificity of neisserial Opa proteins with regard to receptor recognition, these human immunosuppressive effects would not be evident in animal models. Hence, neisserial strains deficient in *CEACAM1* binding activity are used in the development of OMVs-based human vaccines of the invention.

Pathogenic and commensal *Neisseriacae* are each capable of expressing Opa proteins which are, in the vast majority of instances, recognized by host *CEACAM1.* We have demonstrated the presence of such proteins in OMV preparations, and further, that these species are capable of interacting with human lymphocytes in a manner inducing immunosuppression.

We have noted significant differences in response to OMVs derived from N. *meningitidis,* (Nm) *N. lactamica* (Nl) and *N. gonorrhoeae* (Ng) such that, in analysis of proliferation, inhibition mediated by Nm OMVs was dependent on prior lymphocyte activation, whereas challenge with *CEACAM1* reactive Ng OMVs inhibited proliferation in the absence of lymphocyte prestimulation. We also consider it noteworthy that Nl OMVs (selected according to the invention to be Opa-free) did not induce lymphocyte proliferation while Opa-ve (or otherwise *CEACAM1* non-reactive) Ng OMVs induced proliferation relative to unchallenged lymphocytes.

The invention also differs surprisingly from a previous study in which infection with intact bacteria (expressing Opa) had little appreciable effect on cell death. In the invention, compositions prepared from those bacteria, e.g. OMVs, did affect cell death.

According to the invention, avoiding the use of *CEACAM1* reactive Opa, (in the context of an OMV) inhibits associated functions and has significant benefits in protective efficacy of all meningococcal vaccines. Commercially available (meningococcal) OMV vaccines are prepared from clinical isolates and as such are likely to be Opa⁺ in the overwhelming majority of cases. In addition, >95% of expressed meningococcal Opa variants are recognised by *CEACAM1* and consequently, it is highly probable that current vaccine preparations induce immunosuppressive effects through ligation of *CEACAM1.* Following the invention, vaccine "parent strains" are screened for Opa expression, and in particular, for *CEACAM1* reactive species. Analysis of this type enables selection of Opa(-) or otherwise *CEACAM1* non-reactive bacterial isolates, and consequently, a relative enhancement in immune response to OMVs obtained therefrom.

Both the meningococcus and gonococcus are obligate human pathogens and, consistent with this, Opa variants do not recognise murine or other *CEACAM1,* as already mentioned. Consequently, CEACAM mediated immunosuppression would not be evident in the animal models typically used to assess vaccine efficacy. Opa is an important neisserial pathogenicity determinant, and exclusion of this species may unduly restrict the potential immunogenicity of an OMV based (or other) vaccine. As a result, in accordance with specific embodiments of the invention a native non-immunosuppressive Opa, or a mutant protein modified to this end, is included in an OMV- based composition. Given the genetic plasticity typical of the *Neisseriacae,* and the availability of a complete meningococcal genome sequence antigenic selection and/or genetic manipulation can be used to engineer an optimised OMV preparation derived from the pathogenic *Neisseriacae.* Further, given the comparatively innocuous nature of the commensal species *N. lactamica* coupled with observations of heterologous protection afforded by OMVs from this species and, potentially, the expression of novel heterologous antigens in this context non-*CEACAM1* reactive (or otherwise Opa^{-ve}) *N. lactamica* is also suitable for an improved *N. meningitidis* vaccine.

The invention is now illustrated in the following examples, with reference to the accompanying drawings in which:-
Fig 1 is a scanning electron micrograph illustrating intact diplococci and isolated outer membrane vesicles (OMVs);
Fig 2 shows Opa expression pattern and *CEACAM1* binding properties of neisserial OMVs;
Fig 3 shows proliferation of T lymphocytes in response to neisserial OMVs;
Fig 4 is a FACS analysis of CD69 expression by Jurkat cells; and
Fig 5 shows apoptotic mortality among IL2 stimulated Jurkat cells in response 16 h challenge using gonococcal OMVs.

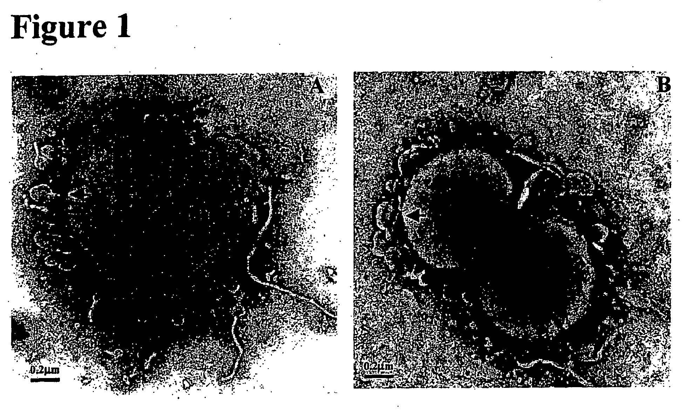

In more details, figure 1 shows a scanning electron micrograph illustrating intact diplococci and isolated outer membrane vesicles (OMVs). *N. meningitidis* (A), and *N. lactamica* (B) each have closely associated naturally occurring membrane "blebs" (filled arrows).

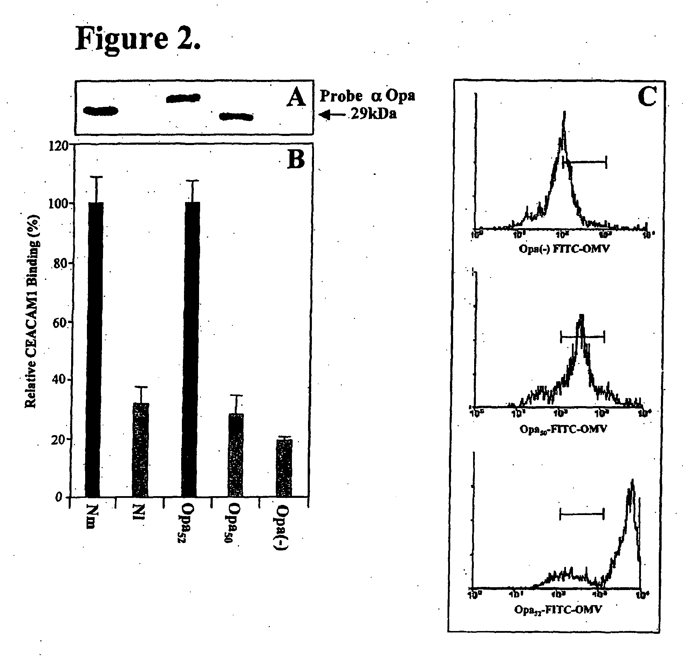

Figure 2 shows Opa expression pattern and *CEACAM1* binding properties of neisserial OMVs. (A) Immunoblot probed using_Opa protein specific mAb illustrating the presence of an immunoreactive Opa variant in Nm-OMVs, but not in comparable Nl-OMVs; and appropriate Opa phenotypes in OMVs derived from isogenic gonococcal strains. (B) : ELISA assay quantifying interactions between neisserial OMVs (10µg total protein) and soluble *CEACAM1-*Fc. OMVs which recognize *CEACAM1* are shown as black bars. In each instance, error bars indicate + 1 SD (n = 3).

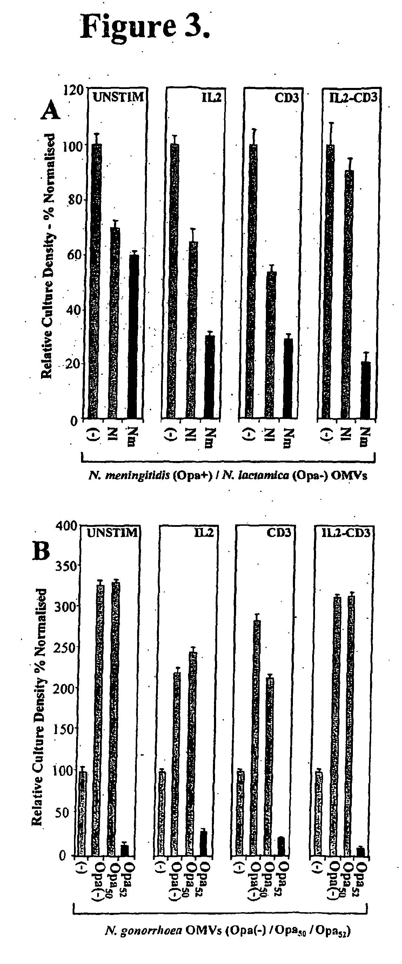

Figure 3 shows proliferation of T lymphocytes in response to neisserial OMVs. Jurkat T lymphocytes were cultured in the presence of 10,000 U/IL2 and/or 1µg anti_CD3∈ Ig and were then challenged using OMVs derived from either *N. meningitidis* (Nm), or *N. lactamica* (Nl) (A); or *N. gonorrhoeae* expressing defined Opa variants (B). Challenge with *CEACAM1* reactive OMVs are shown as black bars. In each instance, error bars indicate + 1 SD (n = 6). Statistical analysis of these data indicate that NL and NM data differ with a confidence interval of p< 0.010 coincident with lymphocyte prestimulation and p = 0.06 in the absence of prestimulation. Similar interrogation of gonococcal OMV data demonstrated that Opa₅₂challenge data differed from comparable challenge data with a confidence interval of p< 0.0002.

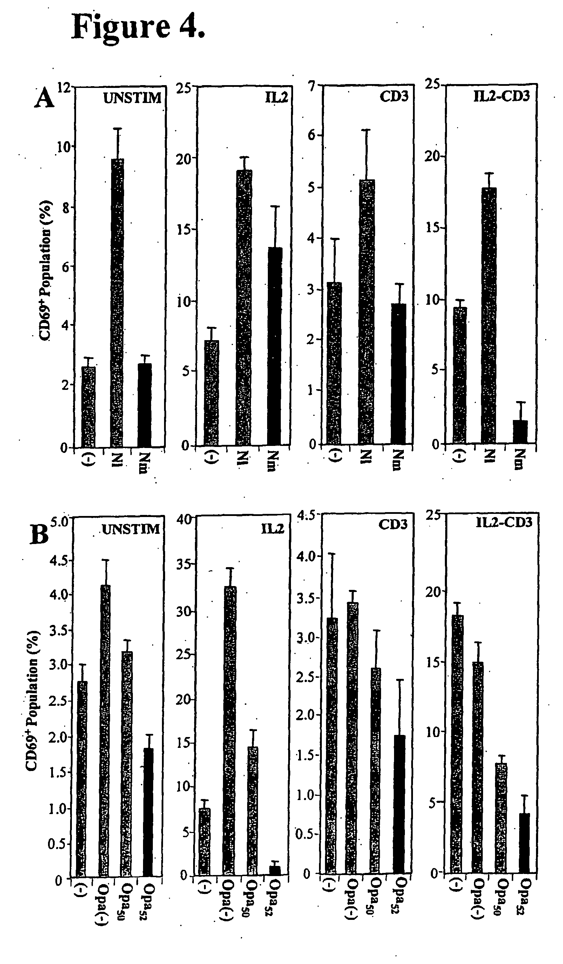

Figure 4 is a FACS analysis of CD69 expression by Jurkat cells. Matched cell populations were prestimulated as indicated (using 10,000 U/IL2 and or 1µg anti_CD3∈ Ig) and were challenged with OMVs derived from *N. meningitidis* (Nm) or *N. lactamica* (Nl), (A), or using OMVs derived from *N. gonorrhoeae,* expressing defined Opa variants (B). In each instance, error bars indicate + 1 SD (n = 3 groups of 1 x 10⁵ events). Statistical analysis established that Nm and Nl data differed with a confidence interval ranging from p = 0.01 to p < 0.0001 with the most robust differences being evident either in unstimulated lymphocytes, or those prestimulated with IL-2 and anti_CD3∈ Ig. Opa₅₂ challenge data differed from comparable data with confidence intervals ranging from p = 0.0037 - p < 0.0001

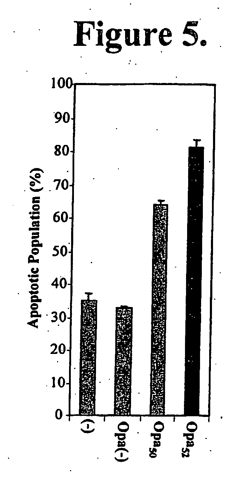

Figure 5 shows apoptotic mortality among IL2 stimulated Jurkat cells in response 16 h challenge using gonococcal OMVs. OMV challenge resulted in a dose dependent increase in apoptosis with the largest effects coincident with challenge using Opa52 OMV which react with *CEACAM1.* Challenge using Opa50 OMVs had an intermediate effect, and challenge using Opa(-) OMVs had a minimal effect on levels of apoptosis. In, each instance these data are representative of 1 x 10⁶ lymphocytes interrogated by flow cytometry. Data are representative of three experiments.

### EXAMPLE 1

### Materials and Methods

### Cell Lines and Tissue Culture Techniques

Jurkat (CD4⁺) human T lymphocytes (ATCC#CRL-10915) have been described previously {Nagasawa, Howatson, et al.1981 ID: 2798} and were routinely maintained in RPMI 1640 medium (Invitrogen; Burlington, Ontario) supplemented with 10% heat inactivated fetal bovine serum and 4 mM GlutaMAX (Invitrogen), referred to as RPMI-G. Cells were cultured at 37°C in 5% CO₂ humidified air. Where appropriate, Jurkat cells were stimulated for 48 h using the indicated concentrations of recombinant human IL-2 (Pharmingen; Mississauga, Ontario) prior to OMV challenge. Challenges of this type were carried out in RPMI-G supplemented with 5 U/ml benzonase endonuclease (Sigma; Oakville, Ontario) and 5% (v/v) phosphate-buffered saline (pH 7.4; PBS) with 1 mM MgCl₂ and 0.5 mM CaCl₂ (referred to as PBS/Mg/Ca). In some instances stimulation via the T cell receptor was induced by exposure to the human CD3∈-specific monoclonal antibody UCHT1 (Pharmingen), which was subsequently cross-linked using Fab₂ fragments of sheep antimouse IgG (Sigma; 3 µg/ml).

COS-7 African Green Monkey kidney cells (ATCC#CLR-1651) were maintained in DMEM (Invitrogen) supplemented with 10% heat inactivated FBS (Cansera; Etobicoke, Ontario), 100 units/ml penicillin/streptomycin, 1 mm glutamax, 1 mM sodium pyruvate and 1 mM non-essential amino acids (Invitrogen) at 37°C in 5% CO₂ humidified air.

### Bacterial Strains

*Neisseria meningitidis* strain K454 and *Neisseria lactamica* strain Y92 1009 isolates were obtained from the Meningococcal Reference Unit, Manchester, UK. Gonococcal strains constitutively expressing specific Opa variants of *N*. *gonorrhoeae* strain MS 11 have been described previously, and were generously supplied by Dr. T.F. Meyer (Max-Planck-Institut für Infektionsbiologie, Berlin, Germany). Opa variants were expressed in the background of MS 11 strain N279 which does not express pilin and has a deletion in *OpaC₃₀* locus encoding this strain's only HSPG receptor-specific Opa variant. *N. meningitidis* and *N. lactamica* were grown from frozen stocks on Mueller Hinton Agar (Difco Labs; West Molesey, Surrey, UK) and *N. gonorrhoeae* strains were grown from frozen stocks on GC agar (Difco; Oakville, Ontario), supplemented with 1% (v/v) Iso VitaleX^{™} enrichment (BBL^{™}; Becton Dickinson, Cockeysville, MD). All bacterial strains were cultured at 37°C in 5% CO₂ humidified air and were sub cultured daily, using a binocular microscope to monitor Opa phenotype. Opa expression and variant-type were routinely confirmed by SDS-PAGE (10%) with resolved proteins either being stained using Coomassie Brilliant Blue or subjected to immunoblot analysis using the Opa specific monoclonal antibody 4B12/C11, which reacts with all known Opa variants and was generously provided by Dr. M. Achtman (Max-Planck-Insitut für Infektionsbiologie, Berlin, Germany).

### Preparation and physical characterization of OMVs

OMVs were prepared from *N. meningitidis* and *N. lactamica* isolates. Overnight liquid cultures were prepared in Franz medium, and pelleted by centrifugation at 1000 x g. Bacteria were resuspended in OMV buffer containing 0.15 M NaCI, 0.05 M Tris-HCI, 0.01 M EDTA (pH 7.5). Bacterial suspensions were then heated to 56°C for 30 minutes, and resultant extracts then clarified by centrifugation at 25,000 x g for 20 min. Recovered supernatants were centrifuged at ~100,000 x g for 2 h. The final OMV-containing pellet was washed twice, resuspended in PBS and stored at -80°C.

Gonococcal OMVs were prepared from recombinant gonococcal strains with defined Opa phenotypes. Bacteria were passaged overnight on solid medium (as described above), and near stationary phase liquid cultures were prepared in modified Brain Heart Infusion (Difco) containing 10 mM LiCI, 1 mM MgCl, 2 mM CaCl, 50 mM Hepes, 1 % D-Glucose (pH 7.2), cultured at 37°C in 5% CO₂ humidified air with rapid shaking. Thereafter, incubation was continued for an additional 2 h at 40 °C with rapid shaking. Bacteria were removed by centrifugation at 1000 x g for 20 min, and resuspended in PBS containing 0.05% (w:v) sarkosyl (Bioshop Canada, Inc; Burlington, Ontario) and 0.05% sodium deoxycholate (w:v) (Bioshop Canada, Inc). Resuspended cells were incubated at 56°C for 30 min. with gentle mixing and then chilled on ice. Bacterial suspensions were then extracted by using a Wheaton homogenizer and were then sonicated on ice (5 x 10 s pulses). Extracts were clarified by centrifugation at 25,000 x g for 20 minutes, and the resulting supernatant centrifuged at 100,000 x g for 2 h. The final pellet, which contains OMVs, was washed twice, resuspended in PBS, extruded through a 0.22 µm syringe filter, and stored at-80°C. OMV sterility was tested by inoculation onto GC agar and incubation as described above. The size distribution of OMVs was determined either by scanning electron microscopy (*N. lactamica* and *N. meningitidis* OMVs) by comparative FACS analysis (gonococcal OMVs). Estimates of relative surface area were calculated based on the relationship S = 4πr² where S represents the surface area of a sphere, and r represents the radius of that sphere.

### Construction and Expression of CEACAM1-Fc Fusion Proteins

An expression vector encoding the *CEACAM1* amino-terminal-domain fused to the Fc portion of human IgGl was generously provided by Drs. O. Mandelboim and G. Markel (Hadassah Medical School, Jerusalem, Israel). Recombinant *CEACAM1-Fc* protein was expressed in COS-7 cells were transiently transfected using FuGENE6 reagent (Roche Molecular Biochemicals, Indianapolis, Indiana, USA) according to the manufacturer's specifications. Cell culture supernatant was harvested 48-72 h after transfections and was clarified by centrifugation at 1000 x g for 20 minutes at 4°C. Clarified supernatant was then filtered using a vacuum-driven disposable filtration system (Stericup 0.22 µm, Millipore; Nepean, Ontario), and concentrated using a 10 kDa-cut-off polyethersulfone ultrafiltration concentrator (Millipore). The fusion protein was then purified using Protein A-Sepharose (Sigma). Elution from this matrix was performed using 0.2 M glycine/HCl (pH 2.5), with aliquots being recovered directly into collection tubes containing 100 µl 1 M Tris (pH 9.0) to neutralize the samples. Purified eluate was dialyzed against PBS at 4°C and then concentrated to less than 1 mL with Ultrafree Biomax centrifugal filters (Millipore). The receptor function and specificity of *CEACAM1-*Fc fusions was assessed by association with isogenic gonococcal strains possessing specific Opa protein variants

### Determination of Opa receptor specificity.

Interactions between Opa variants and *CEACAM1* were characterised by ELISA. Initially, the protein content of each OMV preparation was determined using the BCA assay system (Pierce Chemical Company, Rockford, Illinois), and samples containing equal amount of total protein were immobilized on 96 well microtitre plates (Coming Corporation; Midland Michigan, USA). Each OMV was applied in triplicate serial doubling dilutions, and then exposed to a standard concentration of the *CEACAM1-*Fc fusion protein. Bound protein was detected using protein A-conjugated horseradish peroxidase, and visualized using the OPD colorometric system (Sigma). OPD-associated signal was quantified by specrophotometric analysis at 450 nm.

### Cytometric Analyses.

Jurkat activation was assessed by quantifying expression of the well-characterized T cell activation marker CD69. Cells were infected and/or challenged as described above, and after 16 h samples were probed using anti-human CD69 monoclonal antibody (clone FN50) conjugated to allophycocyanin (Pharmacia; Mississauga, Ontario). Cells were then fixed in paraformaldehyde (3.7%) and CD69-associated fluorescence was assessed using a FACScalibur flow cytometer (Becton Dickinson, Oakville, Ontario). Cell death was characterized and quantified using the Annexin-V-FLUOS / Propidium iodide staining kit (Boehringer Mannheim, Laval, Quebec), thereby allowing proportional comparison of live, apoptotic and necrotic populations by flow cytometry.

### Analysis Of Lymphocyte Proliferation.

Parallel, density-matched, cultures of Jurkat cells were challenged in RPMI-G + 5% PBS/Mg/Ca using OMVs at the indicated concentration. Gentamycin (100 µg/ml; Bioshop) was added 2 h after the commencement of infection and was maintained throughout the subsequent incubation period to prevent bacterial overgrowth. Some cells were cultured in the presence of 10,00 U recombinant human IL-2 (Pharmingen) and stimulation via the T cell receptor was induced by exposure to 1 µg/ml mouse anti-human CD3∈ IgG (clone UCHT1; Pharmingen). Jurkat proliferation was assessed by direct counting using a Levy double hemocytometer 72 h following infection. In each instance, proliferation was assessed using a standardized counting pattern and no less than six fields were counted for each sample.

### Results

*Opa protein presented by neisserial OMVs.* Neisseria sp. naturally overproduce, evaginate and release outer membrane vesicles (OMVs) or "blebs" (Fig. 1A-B). OMVs obtained from *N. meningitidis* (Nm-OMV) and *N. lactamica* (Nl-OMV) are currently being assessed for efficacy as a vaccine providing protection against meningococcal disease. OMVs were also prepared from isogenic strains of *N. gonorrhoeae* expressing well-defined Opa variants, including the transparent (Opa⁻) strain N302, the heparan sulfate proteoglycan (HSPG) receptor-specific Opa₅₀-expressing N303, and the CEACAM-specific Opa₅₂-expressing N309. Gonococcal OMVs (Ng-OMV) were of similar size to those in the vaccine preparations, as determined by comparative flow cytometric analysis (data not shown).

Equivalent amounts of each OMV, as determined by protein content, were grossly characterized by SDS-PAGE (data not shown). Consistent with the phase and antigenic variability of neisserial outer membrane proteins {Nassif, Pujol, et al. 1999 ID: 2839}, considerable variation was apparent between the various preparations (data not shown). Immunoblots probed using the Opa-specific monoclonal antibody (mAb) that cross-reacts with all known Opa variants illustrated the presence of a single Opa variant in Nm-OMVs, while none was apparent in the Nl-OMV preparation (Fig 2A). Appropriate Opa phenotypes were confirmed in OMVs derived from the recombinant gonococcal strains (Fig 2A). The OMVs containing non-*CEACAM1* reactive Opa and the Opa-free Nl-OMVs were therefore selected as suitable for the invention and were compared with Opa-containing Nm-OMVs. Where expressed, Opa variants were present in comparable concentrations, indicating no gross differences with regard to the density of Opa proteins per unit OMV protein.

Equal amounts of OMVs were exposed to soluble *CEACAM1-*Fc fusion proteins in an ELISA assay. This analysis indicated that the Opa⁺ Nm-OMVs, and those derived from Opa₅₂-expressing gonococci bound the soluble *CEACAM1*-Fc fusion at levels significantly (p<0.0001) greater than either the Nl-OMVs, which lack Opa proteins, or those derived from either transparent (Opa⁻) or Opa₅₀-expressing gonococci. This indicated that the *CEACAM1*-specific binding function of gonococcal Opa₅₂ was maintained in OMV preparations, and demonstrated that the Opa protein variant present in Nm-OMVs recognizes *CEACAM1.* Differences in receptor recognition remained significant with OMV protein concentrations ranging from 0.1 µg/ml to 10µg/ml (Fig. 3 and data not shown).

*CEACAM-specific OMVs inhibit lymphocyte activation.* Previously, we have observed that gonococci expressing *CEACAM1* -specific Opa variants suppress CD4⁺ T cell proliferation in response to activating stimuli. Given that Nm-OMVs differed from Nl-OMVs with respect to Opa phenotype (Fig 2A), and *CEACAM1* binding (Fig. 2B), we tested whether there was a difference in lymphocyte response to these standard vaccine preparations. The degree of inhibitory effect was determined by the presence of a *CEACAM1* binding Opa variant, and further, was influenced by the type of stimulation applied to the lymphocytes. A small difference in cell culture growth (p<0.06) was apparent when Nl-OMV and Nm-OMV were applied to unstimulated Jurkat CD4⁺ T cells. However, when lymphocytes were stimulated using the cytokine IL-2 and/or T cell receptor ligation prior to OMV challenge, the Opa associated differences increased in significance (p<0.01) (Fig. 3A). To confirm that *CEACAM1* binding was sufficient to confer this inhibitory effect, we compared the effect of OMVs derived from isogenic gonococcal strains expressing defined Opa variants with distinct receptor specifities. In contrast to effects observed following challenge using the Nl- or Nm OMVs, challenge using either Opa or Opa₅₀-containing OMVs stimulated proliferation of primary CD4+ T lymphocytes. This is consistent with previous observations made using intact bacteria (rather than OMVs) as the challenge species. However, Opa₅₂-expressing OMVs potently suppressed Jurkat cell proliferation (Fig. 3B), confirming that this *CEACAM1-specific* Opa variant retains both its receptor binding and co-inhibitory functions in this context.

Reduced lymphocyte proliferation could result from a slower rate of cell division among the entire population and/or a reduction in the proportion of cells being stimulated to divide. To determine the proportion of cells that become activated in the presence of various OMV preparations, we quantified expression of the activation marker CD69 by Jurkat T cells. Parallel density-matched lymphocyte populations were pre-stimulated, and then challenged with various OMV preparations. Exposure to *CEACAM1*-reactive OMV s consistently reduced the proportion of lymphocytes expressing CD69 (Fig 4A and B). In the case of the two vaccine preparations, the number of activated Jurkat cells in response to Nm-OMVs was consistently lower than that observed with Nl-OMVs, and was either comparable to (unstimulated and anti-CD3∈-containing samples) or lower than (anti-CD3∈-containing samples) parallel samples not exposed to OMVs (Fig. 4A). Challenge with gonococcal OMVs (Fig. 4B) demonstrated a consistent pattern of cellular activation such that, in all conditions tested, the presence of OMVs containing the CEACAM-specific Opa₅₂ was coincident with reduced cellular activation. However, in this instance, levels of activation were marginally reduced by exposure to Opa⁺ OMVs (irrespective of receptor specificity) but were, in all instances, more significantly inhibited by exposure to *CEACAM1* reactive OMVs (p<0.001).

*Neisserial OMVs induce apoptosis in* Jurkat cells. To quantify and directly compare the influence of Opa protein expression on cell death, we monitored apoptosis and necrosis in Jurkat cell cultures exposed to various dilutions of gonococcal OMVs (Fig. 5). While cellular necrosis was consistently below 5% irrespective of challenge conditions (data not shown), we observed a dose-dependent increase in apoptosis in response to all three OMV preparations. Apoptosis was clearly higher in response to OMVs containing Opa proteins, suggesting that the bulk of cell death correlated with attachment of OMVs to the lymphocyte surface. Opa₅₂-containing OMVs induced greater apoptosis than those containing Opa₅₀, indicating that some part of this effect was dictated by receptor-specific effects. These results contrast those in which intact gonococci were used as the challenge species, as we have observed no significant induction of either apoptosis or necrosis in response to infection by transparent (Opa⁻), Opa₅₀- or Opa₅₂-expressing bacteria.

### Example 2

### EMS Mutagenesis Protocol

The protocol is as follows:
Grow *N.meningitidis* to an optical density at 600nm (OD₆₀₀) of 0.7 in Franz medium. Transfer 6ml to a 15-ml conical tube; spin down for 5 min at 2,000g.
Wash the cells twice with 10ml of ethyl methanesulfonate (EMS) mutagenesis buffer.
Resuspend the washed cell pellet in 12ml of the EMS mutagenesis buffer.
Dispense the cell suspension in four 2.4ml aliquots into 15-ml conical tubes. In a fumehood, add 62.5µl of EMS (Sigma catalog number M0880) to each aliquot of prepared cell suspension and vortex thoroughly (EMS takes a while to go into solution).
Incubate samples for 0, 1, 1.25 and 1.5 h at 37°C, which should give roughly 100, 80, 50 and 20% survival respectively.
To process samples add 10ml of EMS stop solution; spin down the cells: wash once with 10ml of EMS stop solution; wash the cells once with 10ml of medium; resuspend the washed pellet in 2.5ml of medium and store the cell suspension at 4°C.
To determine the viable-cell titer of mutagenized samples, sonicate each sample five times with a Braun Sonic U sonicator set at B070. Serially dilute and plate in triplicate on selective medium. Incubate plates at 37°C.
To plate out mutagenized cells for single colonies, filter each sonicated sample though 5mm pore filters. The filtration step is necessary in order to obtain a good single cell suspension; however about 95% of total cells are lost during filtration due to removal of clumps of cells. Serially dilute filtered samples and plate on selective medium. Incubate plates at 37°C.
Screen the colonies for bacteria which express *CEACAM1*-non reactive Opa proteins. Subsequently screen for such Opa proteins that induce antibodies which bind native, *CEACAM1*-reactive Opa.

### EMS Mutagenesis buffer

4.23 ml 1 M NaH₂P0₄
5.77 ml 1 M Na₂HP0₄
0.5ml 20% Tween 80
H₂O to 100 ml

### EMS Stop solution

5% sodium thiosulfate, 0.1% Tween 80, filter sterilized, 100 ml (make fresh)

### Example 3

### NTG Mutagenesis

The protocol is as follows:
Grow *N. meningitidis* to an optical density at 600nm (OD₆₀₀) of 0.8 in Franz medium. Transfer 50ml to a 50-ml conical tube; spin down for 15 min at 2,000g.
Discard the supernatant and resuspend the pellet in 5ml of medium.
Aliquot 1ml of cells to individual 15ml round-bottom tubes (Falcon cat. No. 2059) and add nitrosoguanidine (NTG) to a final concentration range of 0 to 1,000mg/ml.
Incubate the cell suspension at 37°C with shaking for 1h.
Wash each aliquot of cells twice with 10ml of medium and finally resuspend the pellet in 1 ml of medium.
Make serial dilutions of each reaction and plate in duplicate to get colony counts for determination of the killing curve.
Repeat the mutagenesis with the concentration ofNTG that yields 90% killing in the above conditions.
Screen the colonies for bacteria which express *CEACAM1-*non reactive Opa proteins. Subsequently screen for such Opa proteins that induce antibodies which bind native, *CEACAM1*-reactive Opa.

### Example 4

### Generation of an Opa knock-out mutant

Multiple technologies exist by which to mutate expression of Opa protein variants from the three or four Opa alleles present in *N. meningitidis* (or similar alleles in other Neisseria and/or other bacteria). Briefly, Opa alleles are cloned by virtue of highly conserved regions, in non-coding regions adjacent to regions encoding the Opa peptide itself. Genes cloned in this way are inserted into a suitable plasmid vector and mutagenised using either transposon insertion, or other standard molecular biology techniques (suitable techniques are set out in Sambrook, Fritsch and Maniatis, 1989, Cornelissen et al, 1992; Cornelissen and Sparling 1996, and Boulton et al, 2000).

### Example 5

### Generation of a fragment of Opa that does not bind to CEACAM1

Opa is expressed either in the context of a native organism (ie a Neisseria sp) or a single, cloned, recombinant Opa variant is over-expressed in a heterologous context. In either instance, intact Opa is purified either from bacterial whole cell lysates, purified bacterial membranes or from inclusion bodies, using standard biochemical and / or biophysical techniques, including affinity chromatography and /or selecting filtration and / or gel infiltration chromatography and / or ion exchange chromatography.

Purified Opa is then fractionated by proteolytic digestion using trypsin, chymotrpsin, subtilisin and / or other proteolytic enzymes and / or other chemical treatments. Opa fragments produced in this way are selected for an absence of *CEACAM1* binding, by affinity chromatography, using the *CEACAM1* N terminal - Fc fusion protein described herein, (i.e, by selection of protein fragments which fail to bind this fusion protein). Fragments of this type are used as an antigen in a suitable animal system, in the presence of an adjuvant preparation, and optionally conjugated to a suitable carrier protein. Serum prepared in this way is tested against intact Opa variants by Western blot analysis, and / or whole cell dot blots, and / or cytometric analysis, and / or other reactivity assays. Alternatively, an array of overlapping Opa specific peptides is synthesised *de novo* using standard techniques (Gausepohl et al, 1992), or otherwise expressed from cloned, recombinant nucleic acid sequences encoding specific regions of Opa. In either instance, peptides generated (using either or both techniques) are screened for *CEACAM1* binding according to methods we have defined previously, and can, subsequently, be used as antigens as described.

### Example 6

### Generation of non-CEACAM1 reactive Opa variants

Several naturally occurring gonococcal Opa variants have no *CEACAM1* binding activity, (Gray-Owen et al., 1997) and have been evaluated in terms of immunomodulatory effects. It is also established that, of the four naturally occurring meningococcal Opa variants at least one variant is similarly devoid of *CEACAM1* binding activity (Meutzner et al, 2000), and, in addition, commensal Neisseria sp. express Opa variants which do not recognize *CEACAM1* (Toleman, Aho and Virji, 2001).

A given bacterial culture is screened for Opa expression and *CEACAM1* reactivity (or its absence), and the results used to select a phenotype that is non *CEACAM1* reactive. Opa expression is phase variable, and therefore it is preferred that this process of selection be performed on a routine basis. The screening is suitably performed prior to OMV purification. It can also be performed on OMV preparations from Gram negative bacteria.

Alternatively, a bacterium (preferably a neisserial strain) is constructed that is capable of expressing a single Opa of defined phenotype and receptor specificity. This is achieved by replacing a non functional (i.e. mutated) Opa allele, in the bacterial chromosome with a native (i.e. non-mutated) sequence. Alternatively, Opa is cloned in series with one of several well characterized bacterial gene regulation systems; Opa expression is then controlled by manipulation of culture conditions.

The invention thus provides microorganisms, compositions, vaccines, components of vaccines, methods of obtaining the aforementioned and genes encoding the aforementioned, substantially free of Opa that binds *CEACAM1.* These are suitable for use in treatment or prevention of disease caused by Gram negative bacteria, especially Neisseria

## Claims

1. Use of a composition comprising *Neisseria* outer membrane vesicles which contain Opa that does not bind to *CEACAM1* and which are substantially free of Opa that binds *CEACAM1* for the manufacture of a vaccine for treatment or prevention of meningococcal disease, wherein the outer membrane vesicles are from *Neisseria* that have been modified by mutation to express an Opa that does not bind to *CEACAM1.*

2. Use according to Claim 1, wherein the medicament improves stimulation of immune memory or reduces inhibition of T cell function.

3. Use according to Claim 1 or 2, wherein said *Neisseria* is *Neisseria meningitidis.*

4. Use according to any preceding claim, wherein the mutation is by transposon mutagenesis, UV light, EMS mutagenesis or NTG mutagenesis.

5. Use according to any preceding claim, wherein the medicament is for parenteral, intramuscular, trans-dermal, intra-nasal, oral, topical or mucosal administration.

6. Use according to any preceding claim, wherein the outer membrane vesicles comprise a heterologous antigen.

7. Use according to any preceding claim, wherein the composition comprises a carrier.

8. Use according to Claim 7, wherein the carrier is saline solution, sucrose solution, or a pharmaceutically acceptable buffer solution.

9. Use according to any preceding claim, wherein the composition comprises a surfactant.

10. Use according to any preceding claim, wherein said composition comprises an adjuvant.

11. Use according to any preceding claim, wherein said composition comprises microencapsulated outer membrane vesicles.

12. Use according to Claim 11, wherein the microencapsulated outer membrane vesicles comprise a biocompatible polymer shell or core.

13. Use according to Claim 12, wherein the biocompatible polymer shell or core is made from poiylactide-co-glycolide.

14. A method for manufacturing a medicament for treatment or prevention of meningococcal disease, the method comprising:-
(a) obtaining a *Neisseria;*
(b) determining whether the *Neisseria* expresses an Opa protein that binds to *CEACAM1;*
(c) if the *Neisseria* expresses an Opa protein that binds to *CEACAM1,* discarding the *Neisseria* and repeating steps (a) to (c);
(d) retaining the *Neisseria* if it expresses a mutant or variant or fragment or derivative of Opa, wherein the mutant or variant or fragment or derivative does not bind to *CEACAM1;*
(e) raising an antibody to the mutant or fragment or derivative;
(f) determining whether the antibody also binds to an Opa protein that binds to *CEACAM1;* and
(g) preparing a composition comprising the retained *Neisseria* of step (d).

15. A method according to Claim 14, wherein said mutant or variant or fragment or derivative is obtainable by:
(i) obtaining a *Neisseria;*
(ii) carrying out mutagenesis on the *Neisseria;*
(iii) determining whether the *Neisseria* expresses a mutant or fragment or variant or derivative of an Opa protein that does not bind to *CEACAM1;*
(iv) isolating the mutant or variant or fragment or derivative, wherein the mutant or variant or fragment or derivative does not bind to *CEACAM1.*

16. A method according to Claim 15, wherein said mutagenesis is by transposon mutagenesis, UV light, EMS mutagenesis or NTG mutagenesis.

17. A method according to any of Claims 14 to 16, wherein said determining comprises exposing said Opa protein to a *CEACAM1-Fc* fusion protein in an ELISA assay.

18. A method according to Claim 17, wherein said determining further comprises contacting said Opa protein with an Opa-specific monoclonal antibody.

19. A method according to any of Claims 14 to 16, wherein said determining comprises characterizing the interaction between said Opa protein and *CEACAM1* by ELISA.

20. A method according to Claim 15, further comprising:
(v) raising an antibody to the mutant or fragment or variant or derivative or mimic; and
(vi) determining whether the antibody also binds to an Opa protein that binds to *CEACAM1.*

21. A method according to any of Claims 14 to 20, wherein the bacterium is *Neisseria meningitidis.*

22. A method according to any of Claims 14 to 21, comprising preparing an outer membrane vesicle from the retained bacterium.

## Patentansprüche

1. Verwendung einer Zusammensetzung, umfassend Vesikel der äußeren Membran von *Neisseria,* welche Opa enthalten, das nicht an *CEACAM1* bindet, und welche im Wesentlichen frei sind von Opa, das an *CEACAM1* bindet, zur Herstellung eines Impfstoffes zur Behandlung oder Vorbeugung von Meningokokken-Erkrankung, wobei die Vesikel der äußeren Membran von *Neisseria* sind, welche durch Mutation modifiziert wurden, um ein Opa zu exprimieren, welches nicht an *CEACAM1* bindet.

2. Verwendung gemäß Anspruch 1, wobei das Medikament die Stimulation des Immungedächntisses verbessert oder die Inhibierung der T-Zellen-Funktion verringert.

3. Verwendung gemäß Anspruch 1 oder 2, wobei das *Neisseria Neisseria meningitidis* ist.

4. Verwendung gemäß einem vorhergehenden Anspruch, wobei die Mutation durch Transposonmutagenese, UV-Licht, EMS-Mutagenese oder NTG-Mutagenese stattfindet.

5. Verwendung gemäß einem vorhergehenden Anspruch, wobei das Medikament für parenterale, intramuskuläre, transdermale, intranasale, orale, topische oder mukosale Verabreichung ist.

6. Verwendung gemäß einem vorhergehenden Anspruch, wobei die Vesikel der äußeren Membran ein heterologes Antigen umfassen.

7. Verwendung gemäß einem vorhergehenden Anspruch, wobei die Zusammensetzung einen Träger umfasst.

8. Verwendung gemäß Anspruch 7, wobei der Träger Salzlösung, Saccharoselösung oder eine pharmazeutisch verträglicher Puffer-Lösung ist.

9. Verwendung gemäß einem vorhergehenden Anspruch, wobei die Zusammensetzung ein grenzflächenaktives Mittel umfasst.

10. Verwendung gemäß einem vorhergehenden Anspruch, wobei die Zusammensetzung ein Adjuvans umfasst.

11. Verwendung gemäß einem vorhergehenden Anspruch, wobei die Zusammensetzung mikroverkapselte Vesikel der äußeren Membran umfasst.

12. Verwendung gemäß Anspruch 11, wobei die mikroverkapselten Vesikel der äußeren Membran eine(n) biokompatible(n) Polymerschale oder -kern umfassen.

13. Verwendung gemäß Anspruch 12, wobei die/der biokompatible Polymerschale oder -kern aus Polylaktidcoglykolid hergestellt sind.

14. Verfahren zur Herstellung eines Medikaments zur Behandlung oder Vorbeugung von Meningokokken-Erkrankung, das Verfahren umfassend:
(a) das Gewinnen eines *Neisseria,*
(b) das Bestimmen, ob das *Neisseria* ein Opa-Protein exprimiert, welches an *CEACAM1* bindet;
(c) wenn das *Neisseria* ein Opa-Protein exprimiert, welches an *CEACAM1* bindet, das Verwerfen des *Neisseria* und das Wiederholen der Schritte (a) bis (c);
(d) das Erhalten des *Neisseria,* wenn es eine Mutante oder Variante oder ein Fragment oder Derivat von Opa exprimiert, wobei die Mutante oder Variante oder das Fragment oder Derivat nicht an *CEACAM1* binden;
(e) das Aufbauen eines Antikörpers gegen die Mutante oder das Fragment oder Derivat;
(f) das Bestimmen, ob der Antikörper auch an ein Opa-Protein bindet, welches an *CEACAM1* bindet; und
(g) das Herstellung einer Zusammensetzung, umfassend das erhaltene *Neisseria* von Schritt (d).

15. Verfahren gemäß Anspruch 14, wobei die Mutante oder Variante oder das Fragment oder Derivat erhalten werden können durch:
(i) das Erhalten eines *Neisseria;*
(ii) das Durchführen von Mutagenese an dem *Neisseria*;
(iii) das Bestimmen, ob das *Neisseria* eine Mutante oder ein Fragment oder eine Variante oder ein Derivat eines Opa-Proteins exprimiert, welches nicht an *CEACAM1* bindet;
(iv) das Isolieren der Mutante oder Variante oder des Fragments oder Derivats, wobei die Mutante oder Variante oder das Fragment oder Derivat nicht an *CEACAM1* binden.

16. Verfahren gemäß Anspruch 15, wobei die Mutagenese durch Transposonmutagenese, UV-Licht, EMS-Mutagenese oder NTG-Mutagenese stattfindet.

17. Verfahren gemäß einem der Ansprüche 14 bis 16, wobei das Bestimmen das Aussetzen des Opa-Proteins einem *CEACAM1*-*Fc*-Fusionsprotein in einem ELISA-Test umfasst.

18. Verfahren gemäß Anspruch 17, wobei das Bestimmen ferner das Inkontaktbringen des Opa-Proteins mit einem monoklonalen Opa-spezifischen Antikörper umfasst.

19. Verfahren gemäß einem der Ansprüche 14 bis 16, wobei das Bestimmen das Charakterisieren der Wechselwirkung zwischen dem Opa-Protein und *CEACAM1* durch ELISA umfasst.

20. Verfahren gemäß Anspruch 15, ferner umfassend:
(v) das Aufbauen eines Antikörpers gegen die Mutante oder das Fragment oder die Variante oder das Derivat oder Mimetikum; und
(vi) das Bestimmen, ob der Antikörper auch an ein Opa-Protein bindet, welches an *CEACAM1* bindet.

21. Verfahren gemäß einem der Ansprüche 14 bis 20, wobei das Bakterium *Neisseria meningitidis* ist.

22. Verfahren gemäß einem der Ansprüche 14 bis 21, umfassend das Herstellen eines Vesikels der äußeren Membran von dem erhaltenen Bakterium.

## Revendications

1. Utilisation d'une composition comprenant des vésicules de la membrane externe de *Neisseria* qui contiennent de l'Opa qui ne se lie pas à *CEACAM1* et qui sont pratiquement dépourvues d'Opa qui lie *CEACAM1* pour la fabrication d'un vaccin destiné au traitement ou à la prévention de maladies méningococciques, où les vésicules de la membrane externe proviennent de *Neisseria* qui ont été modifiées par mutation pour exprimer une Opa qui ne se lie pas à *CEACAM1.*

2. Utilisation selon la revendication 1, où le médicament améliore la stimulation de la mémoire immunitaire ou réduit l'inhibition de la fonction des cellules T.

3. Utilisation selon la revendication 1 ou 2, où ladite *Neisseria* est *Neisseria meningitidis.*

4. Utilisation selon l'une quelconque des revendications précédentes, où la mutation est par mutagenèse de transposon, lumière UV, mutagenèse à l'EMS, mutagenèse à la NTG.

5. Utilisation selon l'une quelconque des revendications précédentes, où le médicament est pour une administration parentérale, intramusculaire, transdermique, intranasale, orale, topique ou mucosale.

6. Utilisation selon l'une quelconque des revendications précédentes, où les vésicules de la membrane externe comprennent un antigène hétérologue.

7. Utilisation selon l'une quelconque des revendications précédentes, où la composition comprend un support.

8. Utilisation selon la revendication 7, où le support est une solution saline, une solution de saccharose, ou une solution tampon pharmaceutiquement acceptable.

9. Utilisation selon l'une quelconque des revendications précédentes, où la composition comprend un tensioactif.

10. Utilisation selon l'une quelconque des revendications précédentes, où ladite composition comprend un adjuvant.

11. Utilisation selon l'une quelconque des revendications précédentes, où ladite composition comprend des vésicules de la membrane externe micro-encapsulées.

12. Utilisation selon la revendication 11, où les vésicules de la membrane externe micro-encapsulées comprennent une enveloppe ou un coeur de polymère biocompatible.

13. Utilisation selon la revendication 12, où l'enveloppe ou le coeur de polymère biocompatible est fait de poly(lactide-co-glycolide).

14. Procédé de fabrication d'un médicament destiné au traitement ou à la prévention de maladies méningococciques, le procédé comprenant
(a) l'obtention d'une *Neisseria* ;
(b) la détermination si la *Neisseria* exprime une protéine Opa qui se lie à *CEACAM1 ;*
(c) si la *Neisseria* exprime une protéine Opa qui se lie à *CEACAM1,* l'élimination de la *Neisseria* et la répétition des étapes (a) à (c) ;
(d) la rétention de la *neisseria* si elle exprime un mutant ou un variant ou un fragment ou un dérivé d'Opa, où le mutant ou le variant ou le fragment ou le dérivé ne se lie pas *à CEACAM1 ;*
(e) la production d'un anticorps dirigé contre le mutant ou le fragment ou le dérivé ;
(f) la détermination si l'anticorps se lie également à une protéine Opa qui se lie à *CEACAM1 ;* et
(g) la préparation d'une composition comprenant la *Neisseria* retenue de l'étape (d).

15. Procédé selon la revendication 14, dans lequel ledit mutant ou variant ou fragment ou dérivé peut être obtenu par :
(i) l'obtention d'une *Neisseria ;*
(ii) la réalisation d'une mutagenèse sur la *Neisseria* ;
(iii) la détermination si la *Neisseria* exprime un mutant ou un fragment ou un variant ou un dérivé d'une protéine Opa qui ne se lie pas à *CEACAM1 ;*
(iv) l'isolement du mutant ou du variant ou du fragment ou du dérivé, où le mutant ou le variant ou le fragment ou le dérivé ne se lie pas à *CEACAM1.*

16. Procédé selon la revendication 15, dans lequel ladite mutagenèse est par mutagenèse de transposon, lumière UV, mutagenèse à l'EMS, mutagenèse à la NTG.

17. Procédé selon l'une quelconque des revendications 14 à 16, dans lequel ladite détermination comprend l'exposition de ladite protéine Opa à une protéine de fusion *CEACAM1-*Fc dans un test ELISA.

18. Procédé selon la revendication 17, dans lequel ladite détermination comprend en outre la mise en contact de ladite protéine Opa avec un anticorps monoclonal spécifique de Opa.

19. Procédé selon l'une quelconque des revendications 14 à 16, dans lequel ladite détermination comprend la caractérisation entre ladite protéine Opa et *CEACAM1* par ELISA.

20. Procédé selon la revendication 15, comprenant en outre :
(v) la production d'un anticorps dirigé contre le mutant ou le fragment ou le variant ou le dérivé ou le mimétique ; et
(vi) la détermination si l'anticorps se lie également à une protéine Opa qui se lie à *CEACAM1.*

21. Procédé selon l'une quelconque des revendications 14 à 20, dans lequel la bactérie est *Neisseria meningitidis.*

22. Procédé selon l'une quelconque des revendications 14 à 21, comprenant la préparation d'une vésicule de la membrane externe à partir de la bactérie retenue.
